# FASCICULE DE BREVET EUROPEEN

(11) **EP 3 981 454 B1**
(45) Date de publication et mention de la délivrance du brevet: **28.06.2023**
(21) Numéro de dépôt: 21187891.3
(22) Date de dépôt: 27.07.2021
(51) Int. Cl.: A61M 16/00

(54) **APPAREIL ET INSTALLATION DE FOURNITURE DE GAZ THÉRAPEUTIQUE À UN PATIENT AVEC CONTRÔLE DU DÉBIT**
GERÄT UND ANLAGE ZUR THERAPEUTISCHEN GASVERSORGUNG EINES PATIENTEN MIT DURCHSATZSTEUERUNG
DEVICE AND SYSTEM FOR PROVIDING THERAPEUTIC GAS TO A PATIENT WITH CONTROL OF THE FLOW RATE

(30) Priorité: 06.10.2020 FR 2010172
(43) Date de publication de la demande: 13.04.2022
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: BOULANGER, Thierry, Newark, 19702 (US)
(74) Mandataire: Air Liquide

(56) Documents cités:
- EP-A1- 3 701 992
- WO-A1-99/61090
- WO-A1-2013/138905
- WO-A1-2015/048766
- CN-A- 105 771 049

## Description

L'invention concerne un appareil de délivrance de gaz et une installation de fourniture de gaz thérapeutique intégrant un tel un appareil de délivrance de gaz pouvant être utilisés pour fournir un gaz thérapeutique (i.e. gaz pur ou mélange gazeux) à un patient conscient dans différents lieux de soins, en particulier à l'hôpital, y compris dans le cadre d'inhalations de longue durée, par exemple de plusieurs heures, tout en minimisant les pertes de gaz.

Certaines thérapies nécessitent l'administration à des patients conscients de gaz thérapeutique formé d'un mélange de plusieurs constituants gazeux.

Ainsi, il est connu d'utiliser un mélange équimolaire (50%/50%) de protoxyde d'azote (N₂O) et d'oxygène (O₂) pour amoindrir les états d'anxiété, produire un effet sédatif et/ou atténuer la douleur aigue.

De même, il a été proposé d'utiliser un mélange d'argon et d'oxygène (60 vol.% Ar/40 vol.% O₂), inhalé avant et pendant, voire après, une procédure de thrombectomie mécanique, afin de traiter les accidents vasculaires cérébraux ou AVC.

Si l'administration du gaz par inhalation est généralement courte, c'est-à-dire typiquement de moins de 30 min, des inhalations plus longues, de l'ordre de 1h ou plus, sont parfois nécessaires.

De façon générale, l'inhalation du gaz thérapeutique (i.e. 1 ou plusieurs constituants) se fait via un masque respiratoire, typiquement un masque facial, i.e. naso-buccal, à un patient conscient et ce, de façon soit continue, soit intermittente, c'est-à-dire périodiquement.

Pendant une administration continue, un débit continu de gaz, excédant la ventilation minute du patient (i.e. le volume moyen de gaz inhalé par le patient pendant une minute) est délivré en continu pendant les phases inspiratoires et expiratoires du patient. Pendant les phases inspiratoires, le patient inhale le gaz contenu dans un réservoir déformable, alors que pendant les phases expiratoires, le gaz rempli à nouveau le réservoir déformable pour préparer la phase inspiratoire suivante Un tel appareil est présenté dans le document EP 3701992.

Toutefois, une administration continue présente des inconvénients, notamment :
- la ventilation minute du patient doit être vérifiée régulièrement afin de réajuster le débit de gaz, si nécessaire. Ceci requiert une présence quasi-permanente d'un personnel de santé, ce qui n'est pas possible pour les patients traités à domicile et compliqué en milieu hospitalier où les personnels doivent s'occuper de plusieurs patients à la fois.
- le débit sélectionné doit être supérieur à la ventilation minute du patient afin d'éviter que le réservoir ne se vide dans le temps. De ce fait, le débit est toujours réglé en excès. Ceci engendre des pertes importantes de gaz qui s'échappe à l'atmosphère, sans avoir été inhalé par le patient.
- en cas d'inhalation prolongée, par exemple pendant une intervention chirurgicale de plus de 2h, afin de parer à toute variation de la ventilation minute du patient, le débit continu de gaz est réglé à une valeur conservative, qui est généralement bien supérieure à la ventilation minute moyenne du patient. Les volumes de gaz nécessaires sont donc très importants, ce qui engendre une logistique compliquée et des problèmes de place dans la salle d'opération ou analogue : échanges fréquents des bouteilles de gaz vides par des pleines, stockage des bouteilles vides et pleines, manque d'espace libre pour le stockage...

Une délivrance de gaz intermittente, c'est-à-dire non-continue, est donc souvent préférée. Pour ce faire, on utilise classiquement des dispositifs appelés « valves à la demande ».

Durant les phases inspiratoires, une valve à la demande ou VAD s'ouvre et délivre du gaz thérapeutique proportionnellement à la pression négative générée par les inspirations du patient dans le masque respiratoire équipant le patient, alors que, pendant les phases expiratoires, la VAD se referme et arrête de délivrer du gaz.

Une VAD permet de ne fournir la quantité de gaz dont le patient a besoin, c'est-à-dire exactement sa ventilation minute. Ceci permet de limiter fortement la consommation de gaz et donc d'éviter les inconvénients susmentionnés existants lors d'une administration continue.

Cependant, une VAD présente aussi des inconvénients. Ainsi, elle nécessite une pression négative minimale, relativement importante, pour s'ouvrir et fournir le débit de gaz au patient et, une fois ouverte, un effort inspiratoire conséquent de la part du patient est nécessaire pour inspirer le gaz dont il a besoin.

Pour ces raisons, les VAD ne sont pas indiquées pour certains patients, en particulier les personnes fragiles (e.g. enfants en bas âge, personnes âgées...), donc ne sont pas adaptées actuellement au traitement de certaines pathologies affectant ces types de patients, par exemple ceux atteints d'AVC qui sont majoritairement des personnes âgées.

Par ailleurs, un autre problème existe avec les deux systèmes (i.e. continu ou discontinu) dans le traitement de certaines pathologies. Ainsi, du fait de contraintes d'espace relatives à l'acte de thrombectomie mécanique dans le traitement des AVC, le système d'administration doit être placé loin de la tête du patient et raccordé à ce dernier au moyen de tubulures d'amenée de gaz, qui peuvent atteindre plusieurs mètres de longueur, typiquement jusqu'à 4 mètres, voire plus.

Ces tubulures vont engendrer une résistance supplémentaire à l'inspiration, c'est-à-dire que le patient doit produire une pression négative encore plus importante pour pouvoir inspirer du gaz et subvenir à ses besoins ventilatoires.

Or, en cas de « fuites » au niveau du masque, i.e. défauts d'étanchéité sur le pourtour du masque en contact avec le visage, cette pression négative entraine de l'air ambiant et dilue le gaz inhalé.

Cette dilution non-désirée du gaz thérapeutique par de l'air ambiant est très problématique car elle peut conduire à une baisse d'efficacité notable du médicament gaz, i.e. du gaz thérapeutique, administré au patient.

Ceci n'est pas acceptable au plan médical et il est alors nécessaire de minimiser, autant que possible, cette pression négative pour limiter la dilution du gaz thérapeutique avec de l'air ambient, au risque de ne pas obtenir le bénéfice clinique escompté, c'est-à-dire de ne pas traiter efficacement le patient.

Dans ce contexte, un problème est de proposer un appareil de délivrance de gaz et une installation de fourniture de gaz thérapeutique, i.e. gaz pur ou mélange gazeux, à un patient comprenant un tel un appareil de délivrance de gaz permettant de limiter la consommation de gaz, c'est-à-dire fonctionnant de façon analogue à une valve à la demande, tout en assurant un effort inspiratoire minimal du patient pour garantir son confort respiratoire, y compris pendant des procédures longues (e.g. 1 à 2 h ou plus), et limiter autant que possible la dilution du gaz thérapeutique en cas de fuites au masque, c'est-à-dire d'entrées indésirables d'air ambiant du fait de défauts d'étanchéité.

Une solution selon l'invention concerne un appareil de délivrance de gaz comprenant :
- un passage de gaz interne en communication fluidique avec un réservoir déformable pour alimenter ledit réservoir déformable en gaz thérapeutique,
- un dispositif à vanne agencée sur le passage de gaz interne, en amont du réservoir déformable, pour contrôler le débit de gaz thérapeutique circulant dans le passage de gaz interne, et
- une unité de contrôle à microprocesseur pilotant le dispositif à vanne pour fixer ou ajuster le débit de gaz traversant ledit dispositif à vanne et alimentant le réservoir déformable en gaz thérapeutique, et
   caractérisé en ce qu'il comprend en outre un capteur de pression configuré pour :
   - opérer une ou des mesures de pression de gaz (P_{masque}) au niveau d'un masque respiratoire et
   - fournir à l'unité de contrôle la ou lesdites mesures de pression de gaz (Pmasque),
   et dans lequel l'unité de contrôle est configurée pour :
   - comparer la ou les mesures de pression de gaz (P_{masque}) fournies par le capteur de pression à une valeur-seuil de pression donnée (Pₛₑᵤᵢₗ) et
   - commander le dispositif à vanne pour ajuster le débit de gaz en fonction de ladite comparaison.

Dans le cadre de l'invention :
- le terme « pression » est utilisé pour désigner de façon générale, une pression positive (> 0 bar), nulle (= 0 bar) ou négative (< 0 bar), c'est-à-dire une dépression.
- les pressions sont exprimées en bar ou mbar relatifs.
- le signe « - » devant une valeur de pression signifie que la pression est négative, c'est-à-dire qu'il s'agit d'une dépression (i.e. inférieure à la pression atmosphérique).
- le signe « + » devant une valeur de pression signifie que la pression est positive (i.e. supérieure à la pression atmosphérique).
- le terme « gaz thérapeutique » désigne un gaz à un ou plusieurs constituants ou composés gazeux, c'est-à-dire un gaz 'pur' ou un mélange gazeux.
- dans « unité de commande », le terme « unité » est équivalent aux termes « dispositifs », « appareils », « moyens », « système » ou analogues, et le terme « contrôle » est équivalent aux termes « commande », « pilotage », « traitement » ou similaire.

Selon le mode de réalisation considéré, l'appareil de délivrance de gaz thérapeutique de l'invention peut comprendre l'une ou plusieurs des caractéristiques suivantes :
- lorsque l'unité de contrôle détermine que la pression de gaz (P_{masque}) mesurée dans le masque est inférieure ou égale à la valeur-seuil de pression donnée (Pₛₑᵤᵢₗ), i.e. P_{masque} ≤ Pₛₑᵤᵢₗ, ladite unité de contrôle est configurée pour commander le dispositif à vanne pour augmenter le débit de gaz thérapeutique traversant ledit dispositif à vanne et alimentant le réservoir déformable.
- la valeur-seuil de pression (Pₛₑᵤᵢₗ) est inférieure ou égale à 0 mbar.
- la valeur-seuil de pression (Pₛₑᵤᵢₗ) est inférieure ou égale à -0.25 mbar, de préférence inférieure ou égale à -0.5 mbar.
- la valeur-seuil de pression (Pₛₑᵤᵢₗ) est mémorisée au sein de l'unité de contrôle.
- la valeur-seuil de pression (Pₛₑᵤᵢₗ) est mémorisée par le microprocesseur ou par une mémoire de stockage de données.
- la valeur-seuil de pression (Pₛₑᵤᵢₗ) est réglable.
- le passage de gaz interne comprend un ou plusieurs conduits, tuyaux ou analogue.
- le capteur de pression comprend un capteur de pression différentiel.
- le capteur de pression est relié électriquement à l'unité de contrôle.
- le dispositif à vanne comprend une vanne proportionnelle.
- le capteur de pression est configuré pour fournir à l'unité de contrôle une ou des mesures de pression de gaz (P_{masque}), préférentiellement plusieurs mesures de pression successives, sous forme de valeurs numériques ou de signaux représentatifs de telles valeurs numériques (par ex. des signaux de tensions lesquels valeurs ou signaux peuvent être traités tels quels ou convertis en valeurs numériques par l'unité de contrôle.
- le capteur de pression est relié pneumatiquement, via une liaison pneumatique, tel un conduit flexible, à un masque respiratoire pour réaliser des mesures de pression dans la chambre respiratoire interne du corps de masque.
- il comprend une source d'alimentation électrique de type cordon et prise d'alimentation au secteur (e.g. 110/220 V) et/ou une batterie interne, de préférence rechargeable.
- la source d'alimentation électrique alimente en courant électrique l'unité de contrôle et tous les autres composants de l'appareil présents (en fonction du mode de réalisation choisi) qui nécessitent de la puissance électrique pour fonctionner, par exemple un ou des composants de type écran d'affichage, LED, dispositif d'alarme sonore et/ou visuelle...
- il comprend un boitier externe rigide, par exemple en matériau polymère ou autre.
- l'unité de contrôle, au moins une partie du passage de gaz interne, le réservoir déformable, le capteur de pression et/ou le dispositif à vanne sont agencés dans le boitier.
- le réservoir déformable comprend un ballon flexible ou analogue.
- le réservoir déformable se déforme en fonction de la quantité et/ou la pression de gaz thérapeutique qu'il contient. Il peut donc adopter différents états, stades ou niveaux de remplissage, notamment un stade dit « plein », un stade dit « vide » (i.e. quantité résiduelle minimale de gaz) et des stades intermédiaires correspondant à un remplissage partiel du réservoir (i.e. entre les stades « plein » et « vide »).
- le capteur de pression est configuré ou contrôlé pour opérer des mesures de pression (P_{masque}) a des intervalles de temps donnés, de préférence toutes les 20 msec ou moins, préférentiellement toutes les 10 msec ou moins, voire toutes les 5 msec ou moins.
- l'unité de contrôle est configurée pour piloter le dispositif à vanne, en particulier la vanne proportionnelle, pour ajuster (i.e. fixer ou modifier) le débit de gaz traversant ledit dispositif à vanne en fonction de la comparaison faite par l'unité de contrôle entre la pression mesurée au masque (Pmasque) et la valeur-seuil de pression donnée (Pₛₑᵤᵢₗ) préfixée servant de pression référence, en particulier pour augmenter le débit de gaz thérapeutique traversant le dispositif à vanne et alimentant le réservoir déformable, lorsque l'unité de contrôle détermine que la pression de gaz (P_{masque}) mesurée dans le masque est inférieure ou égale à la valeur-seuil de pression donnée (Pₛₑᵤᵢₗ), i.e. P_{masque} ≤ Pₛₑᵤᵢₗ , avec Pₛₑᵤᵢₗ ≤ 0 mbar, de préférence Pₛₑᵤᵢₗ ≤ -0.25 mbar, de manière à accélérer le remplissage dudit réservoir déformable.
- le réservoir déformable est en matériau flexible de type caoutchouc, silicone ou analogue, par exemple un caoutchouc-silicone LSR NuSil.
- l'unité de contrôle à microprocesseur comprend un ou plusieurs microprocesseurs, de préférence un (ou des) microcontrôleur.
- le (ou les) microprocesseur(s) met en oeuvre un ou plusieurs algorithmes.
- l'unité de contrôle comprend une ou plusieurs mémoires de stockage de données ou autres, par exemple des tables de référence.
- l'unité de contrôle à microprocesseur comprend une carte électronique portant le ou les microprocesseurs, de préférence un ou des microcontrôleurs.
- l'appareil de délivrance de gaz comprend en outre un débitmètre ou capteur de débit agencé dans le passage de gaz interne pour mesurer le débit de gaz circulant dans ledit passage de gaz interne.
- le capteur de débit (i.e. débitmètre) agencé dans le passage de gaz interne, en aval du dispositif à vanne, en particulier la vanne proportionnelle, de manière à pouvoir mesurer le débit de gaz fourni par ledit dispositif à vanne.
- le capteur de débit est agencé en amont du réservoir déformable, de préférence en amont du point de raccordement de la ligne d'entrée d'air.
- le capteur de débit est relié électriquement à l'unité de contrôle et lui fournit les mesures qu'il opère.
- le capteur de débit est un capteur de débit massique ou un capteur de pression différentielle.
- il comprend un dispositif anti-retour agencé dans le passage de gaz interne, en aval du réservoir, de préférence un clapet anti-retour.
- le réservoir déformable a un volume compris entre environ 0.1 et 3 L, mesuré au repos (i.e. pression interne égale à la pression atmosphérique).
- le réservoir déformable a une paroi d'épaisseur comprise entre 0.10 et 0.90 mm, typiquement entre 0.25 et 0.75 mm.
- l'appareil comprend en outre une (ou des) valve unidirectionnelle agencée dans le passage de gaz interne, en particulier en aval du réservoir déformable.
- l'appareil comprend en outre une interface homme-machine (IHM) comprenant un écran de visualisation d'informations, de préférence un écran tactile, et/ou une ou des touches ou boutons de sélection, notamment des touches virtuelles s'affichant sur l'écran tactile, et/ou un dispositif de mise en route, tel un bouton allumé/éteint ou « on/off », et/ou d'autres éléments.
- l'appareil comprend en outre un système d'alarme pour alerter l'utilisateur en cas de problème affectant l'appareil ou le gaz, par exemple un défaut de vanne ou de capteur, une mauvaise composition gazeuse (e.g. mélange hypoxique) ou autres. Le système d'alarme peut comprendre des moyens ou un dispositif d'émission de signaux sonores et/ou visuels.

L'installation de fourniture de gaz thérapeutique à un patient comprenant un appareil de délivrance de gaz selon l'invention et un masque respiratoire, ledit masque respiratoire étant en communication fluidique avec le réservoir déformable et alimenté en gaz thérapeutique par ledit réservoir déformable, et par ailleurs relié pneumatiquement au capteur de pression afin de permettre la réalisation des mesures de pression dans le masque.

Selon le mode de réalisation considéré, l'installation de fourniture de gaz thérapeutique selon l'invention peut comprendre l'une ou plusieurs des caractéristiques suivantes :
- le masque respiratoire est un masque facial (i.e. naso-buccal) couvrant le nez et la bouche du patient, en utilisation, c'est-à-dire lorsqu'il est porté par ledit patient.
- elle comprend en outre une source de gaz thérapeutique raccordée fluidiquement au passage interne de gaz pour alimenter ledit passage de gaz en gaz thérapeutique.
- la source de gaz thérapeutique comprend un ou plusieurs récipients de gaz, notamment des bouteilles.
- la source de gaz thérapeutique comprend un récipient de gaz contenant un mélange gazeux O₂/N₂O, de préférence un mélange équimolaire de O₂/N₂O (i.e. 50 mol.%/50 mol.%).
- alternativement, la source de gaz thérapeutique comprend un récipient de gaz contenant un mélange gazeux O₂/argon, de préférence contenant de 35 à 45% vol.% O₂ et de 55 à 65% vol.% Ar, par exemple un mélange contenant de 38 à 43% vol.% O₂ et de 57 à 62% vol.% Ar, notamment un mélange binaire formé de 40% vol.% O₂ et 60% vol.% Ar.
- alternativement, la source de gaz thérapeutique comprend un premier récipient de gaz contenant de l'argon ou du N₂O, un second récipient de gaz contenant de l'oxygène (O₂) et un mélangeur de gaz alimenté en gaz par lesdits premier et second récipients de gaz, ledit mélangeur opérant un mélange des gaz provenant des premier et second récipients de gaz pour obtenir un mélange gazeux O₂/N₂O ou O₂/argon.
- elle comprend un conduit d'amenée de pression, i.e. une liaison pneumatique, agencé entre le masque respiratoire et le capteur de pression de l'appareil de délivrance de gaz.

L'invention va maintenant être mieux comprise grâce à la description détaillée suivante, faite à titre illustratif mais non limitatif, en référence aux figures annexées parmi lesquelles :
Fig. 1 schématise un mode de réalisation d'une installation de fourniture de gaz selon l'invention,
Fig. 2 schématise un mode de réalisation de l'architecture interne d'un appareil de délivrance de gaz selon l'invention,
Fig. 3 illustre le fonctionnement de l'unité de contrôle de l'appareil de délivrance de gaz de Fig. 2, en particulier les courbes de pression et de débit obtenues au fil du temps, et
Fig. 4 est une comparaison des performances d'un appareil de délivrance de gaz selon la présente invention et de plusieurs autres dispositifs.

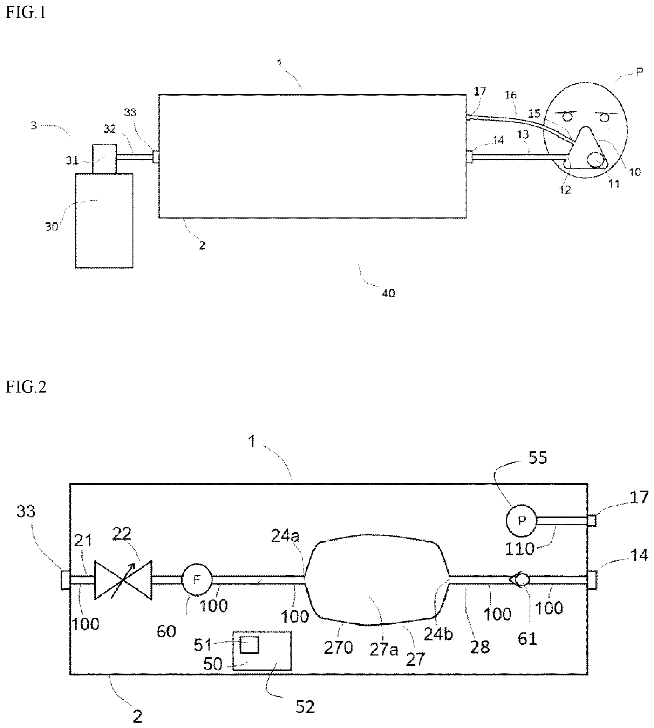

Fig. 1 est une représentation schématique d'un mode de réalisation d'une installation de fourniture de gaz 1 selon la présente invention. Elle comprend un appareil de délivrance de gaz 1 selon l'invention, en particulier celui schématisé en Fig. 2, comprenant un boitier externe 2 formant une carcasse rigide, par exemple en polymère, comprenant les composants internes, notamment un passage de gaz interne, un réservoir déformable, un dispositif à vanne et une unité de contrôle à microprocesseur comme expliqué ci-après.

Une source de gaz thérapeutique 3, telle qu'une bouteille de gaz 30 équipée d'une valve 31, fournit un gaz thérapeutique, c'est-à-dire un gaz ou mélange de gaz à l'appareil de délivrance de gaz 1 via un flexible de raccordement 32, connecté au port d'entrée 33 de l'appareil de délivrance de gaz 1.

Le gaz thérapeutique traverse l'appareil de délivrance de gaz 1, comme expliqué ci-après, pour ensuite être délivré à un patient P par le biais d'une conduite de gaz 13 qui est raccordée fluidiquement à un port de sortie 14 du système d'administration 1. Le gaz est fourni au patient P via une interface ou masque respiratoire 10 alimenté par la conduite de gaz 13, tel un tuyau flexible.

De préférence, l'interface ou masque respiratoire 10 est un masque facial, i.e. un masque naso-buccal, couvrant la bouche et le nez du patient. D'autres interfaces respiratoires pourraient bien entendu convenir.

Le masque facial 10 présente un port d'expiration 11 et un port d'inhalation 12. Le port d'inhalation 12 est connecté fluidiquement à la conduite de gaz 13 qui achemine le gaz. Le port d'expiration 11 comprend préférablement un clapet anti-retour qui dirige et permet l'évacuation à l'atmosphère des gaz lors de l'expiration du patient, c'est-à-dire des gaz expirés riches en CO₂, et empêche par ailleurs l'entrée d'air ambient dans le masque 10 lorsque le patient inhale le gaz thérapeutique, c'est-à-dire pendant ses phases inspiratoires. Le clapet anti-retour comprend une valve unidirectionnelle, tel un disque en silicone reposant sur une surface perforée, qui ne permet au gaz de ne passer que dans un sens, par exemple la valve unidirectionnelle de référence 97351 commercialisée par la société Qosina.

Le masque 10 présente en outre un port de prise de pression 15 connecté fluidiquement à une ligne ou conduit d'amenée de pression 16, tel qu'un tuyau flexible, par exemple un tuyau en silicone de plusieurs mètres, lui-même relié pneumatiquement au capteur de pression 55, via un port de mesure 17 agencé sur le boitier 2 de l'appareil de délivrance de gaz 1. Cette configuration permet au capteur de pression 55 agencé dans le boitier 2 de réaliser des mesures de pression dans le masque 10 afin d'y suivre la pression (i.e. dépression) qui s'y exerce, comme explicité ci-après.

La source de gaz 3 contient un gaz thérapeutique sous pression, par exemple un mélange argon/oxygène, par exemple comprenant 60 vol.% d'argon et 40 vol.% d'oxygène, à une pression maximale de l'ordre de 250 bar. La valve 31 est de préférence un robinet à régulateur de pression intégré ou RDI délivrant le gaz dans le flexible de raccordement 32 à une pression réduite, par exemple de l'ordre de 5 bar. Le régulateur de pression intégré 31 est de préférence protégé par un chapeau rigide (non représenté).

Figure 2 schématise un mode de réalisation de l'architecture interne de l'appareil de délivrance de gaz 1 selon la présente invention qui fait partie de l'installation de fourniture de gaz 40 selon la présente invention, qui est schématisée en Fig.1.

L'appareil de délivrance de gaz 1 comprend une unité de contrôle 50 comprenant un microprocesseur 51 porté par une carte électronique 52 servant à piloter un dispositif à vanne 22, telle une vanne proportionnelle, pour fixer ou ajuster le débit de gaz traversant ledit dispositif à vanne 22, comme expliqué ci-après.

L'unité de contrôle 50 comprend un (ou des) microprocesseur(s) 51, typiquement un (ou plusieurs) microcontrôleur(s), exécutant un (ou plusieurs) algorithme(s) qui reçoit et analyse les mesures fournies par différents capteurs, en particulier par le capteur de pression 55 agencé dans le boitier 2 et relié pneumatiquement au masque 10 par la ligne d'amenée de pression 16.

Un passage de gaz interne 100, par exemple un conduit ou analogue, est arrangé dans le boitier 2 et s'étend entre un port or orifice d'entrée 33 et un port ou orifice de sortie 14 de manière à convoyer le gaz thérapeutique depuis le port d'entrée 33 vers le port de sortie 14, et permettre ensuite son acheminement jusqu'au masque 10, via le conduit flexible 13.

Le dispositif à vanne 22, à savoir ici une vanne proportionnelle, est agencé dans le passage de gaz interne 100, de préférence dans la section amont 21 dudit passage de gaz interne 100. Il est contrôlé par le microcontrôleur 51 de l'unité de contrôle 50 pour modifier le débit de gaz thérapeutique traversant ledit dispositif à vanne 22 et circulant dans le lumen du passage de gaz interne 100 en direction du port ou orifice de sortie 14, comme décrit ci-après.

Différents types de vannes proportionnelles peuvent être utilisés en tant que dispositif à vanne 22, de préférence on choisit une vanne proportionnelle fonctionnant sur une large gamme de débits, par exemple la vanne référencée IMI FAS FLATPROP.

Un capteur de débit 60 est agencé dans le passage de gaz interne 100, en sortie du dispositif à vanne 22, afin de mesurer le débit du gaz thérapeutique délivré par ledit dispositif à vanne 22, typiquement une vanne proportionnelle. Le capteur de débit 60 peut être un capteur de débit massique ou bien basé sur un capteur de pression différentielle.

Le capteur de débit 60 est connecté électriquement à l'unité de contrôle 50 et délivre un signal de débit qui est traité par ladite unité de contrôle 50, typiquement par le microprocesseur 51, préférentiellement un microcontrôleur.

De préférence, un débit volumétrique est obtenu après conversion du signal fourni par le capteur de débit 60 à l'aide d'une table de correspondance spécifique mémorisée dans une mémoire coopérant avec l'unité de contrôle 50. Le capteur de débit 60 peut également être utilisé pour détecter tout défaut de la vanne proportionnelle 22 ou pour déterminer la quantité de gaz, c'est-à-dire le volume, délivré par la source de gaz 3.

Le passage de gaz interne 100 achemine ensuite le gaz vers un réservoir déformable 27, en particulier un réservoir flexible, disposé en aval du capteur de débit 60, et en connexion fluidique avec ledit passage de gaz 100.

Le réservoir déformable 27 comprend une paroi périphérique flexible 270 délimitant un volume interne 27a pour le gaz, formant une poche déformable pour le gaz thérapeutique. Au repos, le volume interne 27a est compris par exemple entre 0,2 et 1 L environ.

Le débit de gaz entre dans le volume interne 27a du réservoir déformable 27 par un orifice d'entrée de réservoir 24a, en communication fluidique avec le passage de gaz intérieur 100. De préférence, les propriétés du réservoir déformable 27 sont telles qu'il est hautement déformable. Par exemple, sa paroi périphérique 270 a une épaisseur comprise entre environ 0,25 et 0,75 mm et est formée d'un silicone flexible biocompatible, par exemple un silicone de la série LSR commercialisée par NuSil.

Le gaz ressort du réservoir 27 par un orifice de sortie de réservoir 24b qui est relié fluidiquement à une section aval 28 du passage de gaz interne 100, se prolongeant jusqu'au port de sortie 14.

Un (ou plusieurs) dispositif anti-retour 61, tel qu'un clapet anti-retour, est agencé dans le passage de gaz interne 100, en aval du réservoir 27, à savoir entre l'orifice de sortie 24b du réservoir 27 et le port de sortie 14 du boitier 2, pour empêcher tout reflux de gaz. Ainsi, les gaz expirés par le patient P sont évacués uniquement par le port d'expiration 11 du masque 10 et ne peuvent pas retourner dans le réservoir 27.

Le clapet anti-retour 61 est de préférence conçu de telle sorte qu'une très faible chute de pression, typiquement inférieure ou égale à 0,2 mbar, est générée à travers lui, quand un flux de gaz le traverse.

Bien entendu, plusieurs clapets anti-retour 61 peuvent également être utilisés à la place d'un seul, par exemple 3 à 5 disposés en parallèle (non représenté).

Afin de permettre de réaliser des mesures de pression dans le masque 10, il est prévu un capteur de pression 55, de préférence un capteur de pression différentielle, dans le boitier 2 de l'appareil 1. Le capteur de pression 55 est configuré pour mesurer des pressions négatives (c'est-à-dire des pressions inférieures à la pression atmosphérique ou dépressions) jusqu'à -5 mb environ.

Le capteur de pression 55 est ici un capteur de pression différentielle qui comprend deux orifices de détection comprenant un premier orifice de détection maintenu aux conditions atmosphériques (c'est-à-dire à la pression atmosphérique, i.e. 1 atm) et un deuxième orifice de détection disposé dans un conduit de mesure 110, raccordé au port de mesure 17 du boitier 2. Le port de mesure 17 est connecté fluidiquement à la ligne d'amenée de pression 16 reliée au masque 10 pour y suivre la pression qui règne dans la chambre respiratoire dudit masque 10. Par exemple, on peut utiliser le capteur de pression différentielle référencé SDP3X disponible auprès de Sensirion.

A des intervalles de temps successifs, par exemple à une fréquence de 5 msec, le capteur de pression différentielle 55 envoie un signal de mesure de pression P_{masque} à l'unité de contrôle 50, lequel signal P_{masque} reflète la pression mesurée dans le masque 10 à l'instant considéré. L'unité de contrôle 50 traite alors ce signal de pression afin de piloter la vanne proportionnelle 22 comme détaillé ci-après, pour ajuster le débit gazeux envoyé au réservoir flexible 27.

Le réservoir flexible 27 présente différents états de gonflage/dégonflage en fonction de la pression de gaz qui y règne, donc de la quantité de gaz qui y est introduite ou soutirée, comprenant au moins :
- un état dit « au repos », dans lequel le volume interne 27a, rempli de gaz, est à pression atmosphérique (i.e. 1 atm).
- un état dit « gonflé », dans lequel le volume interne 27a, rempli de gaz, est à une pression supérieure à la pression ambiante (c'est-à-dire> 1 atm).
- des états de « dégonflage partiel», dans lequel une partie du gaz contenu dans le réservoir en est sorti.

Une source d'alimentation électrique (non montrée) fournit du courant électrique à tous les composants fonctionnant avec de l'énergie électrique, tels que capteurs, unité de contrôle, vannes commandée, interface homme-machine (IHM), écran d'affichage numérique.... Elle peut être disposée dans le boitier 2, par exemple une batterie rechargeable ou comprend un cordon et une prise de raccordement au secteur (110/220V), et éventuellement un convertisseur de courant.

Durant une thérapie avec administration de gaz thérapeutique, le patient P opère une succession d'inspirations et d'expirations pour inhaler le gaz thérapeutique, par exemple un mélange O₂/argon ou N₂O/O₂, et exhaler les gaz riches en CO₂ résultant des échanges pulmonaires.

Afin de faciliter la compréhension du fonctionnement de l'appareil 1, on considère que :
- la pression dans le volume interne 27a du réservoir 27 est égale à la pression atmosphérique, c'est-à-dire que le réservoir 27 est en position de repos.
- le réservoir 27 est rempli du gaz thérapeutique issu de la source de gaz 3.
- le patient entame une inspiration.

Lorsque le patient commence à inspirer, le port d'expiration 11 du masque 10 est fermé et une légère dépression se produit au niveau du port d'inhalation 12 du masque 10. Cette dépression se propage jusqu'au capteur de pression différentielle 55, via respectivement la ligne d'amenée de pression 16, le port de mesure 17 et le conduit de mesure 110. L'information de pression est ensuite transmise par le capteur de pression différentielle 55 à l'unité de traitement 50, en particulier au microprocesseur 51.

Par ailleurs, cette dépression se propage en parallèle dans le conduit 13, le port de sortie 14 et la section aval 28 du passage de gaz interne 100.

Alors que la pression de gaz dans le volume interne 27a du réservoir 27 est égale à la pression atmosphérique (i.e. 1 atm), une pression différentielle positive paraît alors au travers du clapet anti-retour 61 qui permet à une certaine quantité de gaz de passer à travers ledit clapet anti-retour 61 pour répondre à la demande respiratoire du patient.

Autrement dit, un débit gazeux peut s'établir depuis le réservoir 27 en direction du masque 10. Il s'ensuit que le volume interne 27a du réservoir 27 se vide alors et le réservoir 27 se dégonfle, créant à son tour une légère dépression dans le volume interne 27a.

Or, l'unité de contrôle 50 est configurée pour garantir qu'à tout moment, la pression régnant dans le masque 10 soit aussi proche que possible de la pression atmosphérique (i.e. 1 atm), soit 0 mbar relatif. Pour ce faire, l'unité de contrôle 50 pilote la vanne proportionnelle 22 de manière à ce que le débit fourni par ladite vanne proportionnelle 22 soit proportionnel à la pression mesurée P_{masque} dans le masque 10 par le capteur de pression différentielle 55.

A cette fin, le microprocesseur 51 peut par exemple mettre en oeuvre un algorithme du type :
- Si « Pression masque » négative : Débit (L/min) = α * | P |
- Si « Pression masque » positive : Débit (L/min) = 0
   où : α est une constante positive et | P | est la valeur absolue de la pression mesurée dans le masque.

L'unité de contrôle 50 n'agit donc sur la vanne proportionnelle 22 que si la pression régnant dans le masque 10 est négative, c'est-à-dire que la vanne proportionnelle 22 est commandée ou reste en position fermée dès lors que la pression dans le masque 10 devient positive.

En cas de pression négative (i.e. dépression) dans le masque 10, mesurée par le capteur de pression différentielle 55, traduisant une inspiration du patient P, la vanne proportionnelle 22 va être commandée par l'unité de contrôle 50, en particulier par le microprocesseur 51, tel que : Débit (L/min) = α * | P |.

Il apparait alors une proportionnalité entre le débit délivré par la vanne proportionnelle 22 et la pression négative mesurée dans le masque 10 par le capteur de pression différentielle 55. Plus la valeur de pression s'écarte de 0 mb, plus le débit est élevé. A l'inverse, plus la valeur de pression se rapproche de 0 mb, plus le débit est faible. Il est bien entendu que l'algorithme décrit ici à un but illustratif, et que des algorithmes plus sophistiqués tel qu'un contrôle par termes Proportionnel - Intégral et Dérivé (PID) pourraient être implémentés.

En outre, l'appareil de délivrance de gaz 1 peut comprend d'autres éléments, comme une interface homme-machine (IHM) avec écran de visualisation d'informations, de préférence un écran tactile, une ou des touches ou boutons de sélection, un dispositif de mise en route, tel un bouton allumé/éteint, un système d'alarme et/ou d'autres éléments.

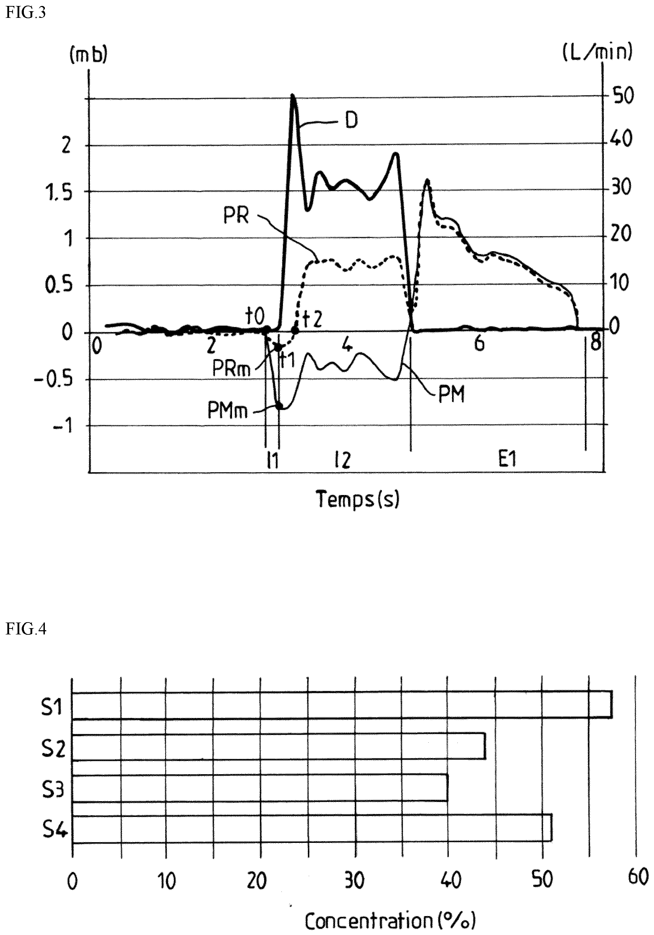

Fig. 3 schématise le fonctionnement de l'unité de contrôle 50, en particulier de l'algorithme mis en oeuvre par le microprocesseur 51 de l'appareil de délivrance de gaz 1, en réponse à une inspiration du patient P.

L'inspiration du patient P se découpe en deux portions distinctes successives I1 et I2 avec I1 correspondant au tout début de l'inspiration. Ainsi, si t0 est l'instant même du début de l'inspiration du patient P, à cet instant la pression relative PR dans le réservoir 27 est nulle, c'est-à-dire à la pression atmosphérique (i.e. 1 atm).

Comme susmentionné, l'inspiration du patient crée alors une dépression dans le masque 10, qui est représentée par la courbe PM. En réponse à cette dépression, l'unité de contrôle 50 va piloter la vanne proportionnelle 22 pour ajuster le débit de gaz thérapeutique afin de limiter la chute en pression dans le masque 10.

Or, comme dans tout système intégrant des éléments électromécaniques, il existe un temps de réponse intrinsèque, c'est-à-dire un délai, en réponse à la manifestation physique qu'est ici la dépression au masque 10.

Pendant cette phase I1, la pression dans le réservoir déformable 27 diminue, signe que ce dernier se dégonfle et qu'une quantité de gaz circule à travers le clapet anti-retour 61 en direction du masque 10 et ce, afin de subvenir à la demande inspiratoire du patient P. Cette diminution de pression dans le réservoir 27 atteint, en t1, une valeur minimale PRm et, de façon similaire, il apparaît une pression minimale PMm dans le masque 10.

Ce temps t1 correspond au moment où l'électrovanne proportionnelle 22 commence à s'ouvrir en réponse à la sollicitation de l'unité de contrôle 50 et à donc délivrer un débit D, marquant la transition en phase I2.

Dans ces conditions, le débit gazeux D va subvenir au besoin du patient P et dans le même temps remplir le réservoir 27 dont la pression PR va croitre jusqu'à redevenir nulle en t2, signe que le réservoir 27 est revenu dans son état de repos, c'est-à-dire pleinement rempli.

Cette augmentation de pression PR dans le réservoir 27 s'accompagne naturellement, dans le même temps, d'un accroissement de la pression PM dans le masque 10, avoisinant ici -0.5 mb.

La portion de la phase I2 ultérieure à t2 voit le réservoir 27 en situation de sur-gonflage car la pression PR est positive, ce qui est parfaitement normal.

En effet, l'unité de contrôle 50 pilote la vanne proportionnelle 22 pour ajuster le débit qui la traverse de manière à ce que la pression PM dans le masque 10 soit la plus proche de 0. Or, les éléments en aval du réservoir 27, notamment la section aval 28 du passage 100, le clapet anti-retour 61 et la ligne 13, créent une résistance à l'écoulement du gaz. En relation à la demande inspiratoire du patient P (c'est-à-dire son débit inspiratoire), cette résistance à l'écoulement est égale à la différence de la pression PR dans le réservoir 27 et de la pression PM dans le masque 10.

En d'autres termes, une pression positive dans le réservoir 27 n'a pour but que de compenser tout ou partiellement la résistance à l'écoulement des éléments susmentionnés afin que la pression négative dans le masque 10 soit la plus proche de 0.

Si l'appareil 1 ne disposait pas d'un mécanisme de délivrance de débit basé sur la pression régnant dans le masque 10, c'est-à-dire si le réservoir 27 se vidait progressivement en réponse à l'inspiration du patient P, alors la dépression dans le masque 10 permettant au patient P de subvenir à ses besoins ventilatoires, serait égale à la somme des résistances à l'écoulement des éléments situés en aval du réservoir 27, incluant le réservoir 27 lui-même.

Enfin, la phase I2 laisse place à une phase expiratoire E1 où le patient expire à travers le port expiratoire 11 du masque 10. Cette expiration génère alors une pression PM positive dans le masque 10 et l'unité de contrôle 50 commande alors la vanne proportionnelle 22 de manière à interrompre la délivrance de gaz, c'est-à-dire le débit. Dans le même temps, le réservoir 27, lui-même sous pression PR positive, se vide progressivement en suivant le profil de la pression PM régnant dans le masque 10.

Le réservoir 27 est indispensable au bon fonctionnement de l'appareil 1. En effet, s'il n'était pas présent, le gaz circulerait dans des éléments rigides, c'est-à-dire non déformables, comme le passage de gaz interne 100 et le conduit 13. Ainsi, lors de la phase I1, avant que la vanne proportionnelle 22 ne s'ouvre, la demande respiratoire du patient ne serait pas satisfaite, entrainant alors un inconfort respiratoire majeur pour le patient. De plus, le réservoir 27, tout au long de la phase inspiratoire, joue un rôle de tampon en atténuant l'effet des variations de la demande ventilatoire du patient P et de la réponse de l'unité de contrôle 50 et de la vanne proportionnelle 22, en réponse à ces variations.

Fig. 4 compare les performances d'un appareil de délivrance de gaz 1 selon la présente invention et de plusieurs dispositifs de l'art antérieur, tels qu'un système à débit continu et une valve à la demande.

Afin d'obtenir des données représentatives, cette comparaison met en oeuvre un banc de test comprenant un « patient électronique », à savoir un dispositif mimant la respiration d'un patient, par exemple le simulateur respiratoire ASL 5000 disponible auprès de Ingmar Medical, permettant de simuler de façon répétable la respiration d'un patient.

Les différents dispositifs testés sont connectés au « patient électronique » au moyen d'un conduit d'acheminement de gaz à orifice calibré simulant une fuite au niveau du masque respiratoire.

La source de gaz thérapeutique fournit un mélange formé de 60% argon et de 40% oxygène (vol.%).

En fonction de la résistance de chacun des dispositifs, donc de la dépression générée par le « patient électronique », il est possible de mesurer la concentration en gaz thérapeutique inhalée par le patient sous l'effet de la dilution avec l'air ambient.

Sur la Fig. 4 :
- S1 est l'appareil de délivrance de gaz 1 de la présente invention, schématisé en Fig. 1 et Fig. 2 ;
- S2 est une valve à la demande, par exemple la VAD de Ease II de GCE ;
- S3 est un système à débit continu, par exemple celui disponible auprès de Intersurgical ; et
- S4 est un appareil de délivrance de gaz analogue à celui de l'invention (i.e. S1) mais dans lequel le système de limitation de la dépression dans le masque a été supprimé (i.e. capteur, ligne d'amenée de pression...).

Les résultats obtenus montrent clairement les limitations des systèmes actuels.

Ainsi, sous l'effet de la fuite simulée, la concentration en argon inhalé par le patient P avoisine 40% (vol.%) pour le système à débit continu S3 et 45% pour la valve à la demande S2, soit respectivement une perte de 20% et 15% en volume d'argon, ce qui ne permet pas de garantir une efficacité du dispositif lors de la délivrance de gaz à un patient puisque la teneur en argon fournie au patient est très inférieure à celle attendue, i.e. 60% vol.

A l'inverse, l'appareil de délivrance de gaz 1 (S1) de l'invention permet de limiter fortement la dilution avec l'air ambient, en maintenant, dans les mêmes conditions d'essai, une concentration de l'ordre de 57 vol.%, soit approximativement la teneur désirée (i.e. 60%), garantissant alors pleinement l'efficacité thérapeutique.

A titre comparatif, l'appareil de délivrance de gaz 1 dépourvu des moyens de limitation de la dépression dans le masque (S4), reste supérieur aux dispositifs existants (S2, S3) mais n'assure cependant qu'une concentration légèrement supérieure à 50 vol.%, ce qui est insuffisant pour garantir une efficacité du traitement à l'argon pour lequel une teneur efficace de 60 vol.% est désirée.

L'appareil de délivrance de gaz 1 selon l'invention répond donc en tout point aux besoins de confort patient et de minimisation de l'impact de fuite en terme de diminution de la concentration des gaz inhalés, assurant ainsi l'efficacité thérapeutique souhaitée.

Ce niveau d'efficacité n'est possible qu'en associant un réservoir déformable 27 et à un asservissement du débit de gaz délivré à la pression mesurée dans le masque 10 par le capteur de pression 55 qui coopère avec l'unité de contrôle 50 à microprocesseur 51.

## Revendications

1. Appareil de délivrance de gaz (1) comprenant :
- un passage de gaz interne (100) en communication fluidique avec un réservoir déformable (27) pour alimenter le réservoir déformable (27) en gaz thérapeutique,
- un dispositif à vanne (22) agencée sur le passage de gaz interne (100), en amont du réservoir déformable (27), pour contrôler le débit de gaz thérapeutique circulant dans le passage de gaz interne (100), et
- une unité de contrôle (50) à microprocesseur (51) pilotant le dispositif à vanne (22) pour fixer ou ajuster le débit de gaz traversant ledit dispositif à vanne (22) et alimentant le réservoir déformable (27) en gaz thérapeutique, **caractérisé en ce qu'**il comprend en outre un capteur de pression (55) configuré pour :
a) opérer une ou des mesures de pression de gaz (P_{masque}) au niveau d'un masque respiratoire (10) et
b) fournir à l'unité de contrôle (50) la ou lesdites mesures de pression de gaz (Pmasque),
et dans lequel l'unité de contrôle (50) est configurée pour :
I) comparer la ou les mesures de pression de gaz (P_{masque}) fournies par le capteur de pression (55) à une valeur-seuil de pression donnée (Pₛₑᵤᵢₗ)
II) et commander le dispositif à vanne (22) pour ajuster le débit de gaz en fonction de ladite comparaison.

2. Appareil selon la revendication 1, **caractérisé en ce que** lorsque l'unité de contrôle (50) détermine que la pression de gaz (P_{masque}) mesurée dans le masque (10) est inférieure ou égale à la valeur-seuil de pression donnée (Pₛₑᵤᵢₗ), (i.e. P_{masque} ≤ Pₛₑᵤᵢₗ), ladite unité de contrôle (50) est configurée pour commander le dispositif à vanne (22) pour augmenter le débit de gaz thérapeutique traversant ledit dispositif à vanne (22) et alimentant le réservoir déformable (27).

3. Appareil selon l'une des revendications précédentes, **caractérisé en ce que** la valeur-seuil de pression (Pₛₑᵤᵢₗ) est inférieure ou égale à 0 mbar.

4. Appareil selon l'une des revendications précédentes, **caractérisé en ce que** la valeur-seuil de pression (Pₛₑᵤᵢₗ) est inférieure ou égale à -0.25 mbar, de préférence inférieure ou égale à -0.5 mbar.

5. Appareil selon l'une des revendications précédentes, **caractérisé en ce que** la valeur-seuil de pression (Pₛₑᵤᵢₗ) est mémorisée au sein de l'unité de contrôle (50), de préférence par le microprocesseur (51) de l'unité de contrôle (50).

6. Appareil selon la revendication 1, **caractérisée en ce que** le capteur de pression (55) comprend un capteur de pression différentiel.

7. Appareil selon l'une des revendications 1 ou 2, **caractérisé en ce que** le dispositif à vanne (22) comprend une vanne proportionnelle.

8. Appareil selon la revendication 1, **caractérisée en ce qu'**il comprend en outre un capteur de débit (60) agencé dans le passage de gaz interne (100) pour mesurer le débit de gaz circulant dans ledit passage de gaz interne (100), de préférence le capteur de débit (60) est agencé en aval du dispositif à vanne (22).

9. Appareil selon la revendication 1, **caractérisée en ce qu'**il comprend en outre un comprend un dispositif anti-retour (61) agencé dans le passage de gaz interne (100), en aval du réservoir déformable (27).

10. Installation de fourniture de gaz thérapeutique (40) à un patient comprenant un appareil de délivrance de gaz (1) selon l'une des revendications précédentes et un masque respiratoire (10), ledit masque respiratoire (10) étant en communication fluidique avec le réservoir déformable (27) et alimenté en gaz thérapeutique par ledit réservoir déformable (27), et par ailleurs relié pneumatiquement au capteur de pression (55).

## Patentansprüche

1. Gasabgabevorrichtung (1) umfassend:
- einen inneren Gasdurchgang (100), der mit einem verformbaren Vorratsbehälter (27) in Fluidverbindung steht, um den verformbaren Vorratsbehälter (27) mit therapeutischem Gas zu versorgen,
- eine Ventilvorrichtung (22), die am inneren Gasdurchgang (100) stromauf des verformbaren Vorratsbehälters (27) angeordnet ist, um den Durchsatz von therapeutischem Gas zu steuern, das im inneren Gasdurchgang (100) zirkuliert, und
- eine Steuereinheit (50) mit Mikroprozessor (51), die die Ventilvorrichtung (22) ansteuert, um den Durchsatz von Gas, das durch die Ventilvorrichtung (22) hindurchgeht und den verformbaren Vorratsbehälter (27) mit therapeutischem Gas versorgt, festzulegen oder anzupassen,
**dadurch gekennzeichnet, dass** sie ferner einen Drucksensor (55) umfasst, der dazu ausgebildet ist:
a) eine oder mehrere Messungen eines Gasdrucks (P_{Maske}) an einer Atemmaske (10) vorzunehmen, und
b) der Steuereinheit (50) die Gasdruckmessung(en) (P_{Maske}) bereitzustellen,
und wobei die Steuereinheit (50) dazu ausgebildet ist:
I) die Gasdruckmessung (en) (P_{Maske}), die vom Drucksensor (55) bereitgestellt werden, mit einem bestimmten Druckschwellenwert (P_{Schwelle}) zu vergleichen
II) und die Ventilvorrichtung (22) zu steuern, um den Gasdurchsatz je nach dem Vergleich anzupassen.

2. Vorrichtung nach Anspruch 1, **dadurch gekennzeichnet, dass**, wenn die Steuereinheit (50) bestimmt, dass der Gasdruck (P_{Maske}), der in der Maske (10) gemessen wird, kleiner als oder gleich dem bestimmten Druckschwellenwert (P_{Schwelle}) ist (d.h. P_{Maske} ≤ P_{Schwelle}), die Steuereinheit (50) dazu ausgebildet ist, die Ventilvorrichtung (22) zu steuern, um den Durchsatz von therapeutischem Gas, das durch die Ventilvorrichtung (22) hindurchgeht und den verformbaren Vorratsbehälter (27) versorgt, zu erhöhen.

3. Vorrichtung nach einem der vorhergehenden Ansprüche, **dadurch gekennzeichnet, dass** der Druckschwellenwert (P_{Schwelle}) kleiner als oder gleich 0 mbar ist.

4. Vorrichtung nach einem der vorhergehenden Ansprüche, **dadurch gekennzeichnet, dass** der Druckschwellenwert (P_{Schwelle}) kleiner als oder gleich -0.25 mbar ist, vorzugsweise kleiner als oder gleich -0.5 mbar.

5. Vorrichtung nach einem der vorhergehenden Ansprüche, **dadurch gekennzeichnet, dass** der Druckschwellenwert (P_{Schwelle}) innerhalb der Steuereinheit (50) gespeichert ist, vorzugsweise durch den Mikroprozessor (51) der Steuereinheit (50).

6. Vorrichtung nach Anspruch 1, **dadurch gekennzeichnet, dass** der Drucksensor (55) einen Differenzdrucksensor umfasst.

7. Vorrichtung nach einem der Ansprüche 1 oder 2, **dadurch gekennzeichnet, dass** die Ventilvorrichtung (22) ein Proportionalventil umfasst.

8. Vorrichtung nach Anspruch 1, **dadurch gekennzeichnet, dass** sie ferner einen Durchsatzsensor (60) umfasst, der im inneren Gasdurchgang (100) angeordnet ist, um den Durchsatz von Gas zu messen, das im inneren Gasdurchgang (100) zirkuliert, der Durchsatzsensor (60) vorzugsweise stromab der Ventilvorrichtung (22) angeordnet ist.

9. Vorrichtung nach Anspruch 1, **dadurch gekennzeichnet, dass** sie ferner eine umfasst eine Rückschlagvorrichtung (61) umfasst, die im inneren Gasdurchgang (100) stromab des verformbaren Vorratsbehälters (27) angeordnet ist.

10. Einrichtung zur Versorgung eines Patienten mit therapeutischem Gas (40) umfassend eine Gasabgabevorrichtung (1) nach einem der vorhergehenden Ansprüche und eine Atemmaske (10), wobei die Atemmaske (10) mit dem verformbaren Vorratsbehälter (27) in Fluidverbindung steht und vom verformbaren Vorratsbehälter (27) mit therapeutischem Gas versorgt wird und darüber hinaus pneumatisch mit dem Drucksensor (55) verbunden ist.

## Claims

1. Gas delivery apparatus (1) comprising:
- an internal gas passage (100) in fluid communication with a deformable reservoir (27) in order to supply the deformable reservoir (27) with therapeutic gas,
- a valve device (22) arranged on the internal gas passage (100), upstream of the deformable reservoir (27), in order to control the flow of therapeutic gas circulating in the internal gas passage (100), and
- a control unit (50) with microprocessor (51) controlling the valve device (22) in order to set or adjust the gas flow passing through said valve device (22) and supplying the deformable reservoir (27) with therapeutic gas, **characterized in that** it further comprises a pressure sensor (55) configured to:
a) perform one or more gas pressure measurements (Pₘₐₛₖ) in a respiratory mask (10) and
b) supply the control unit (50) with said gas pressure measurement (s) (Pₘₐₛₖ),
and in which the control unit (50) is configured to:
I) compare the gas pressure measurement (s) (Pₘₐₛₖ) supplied by the pressure sensor (55) to a given pressure threshold value (P_{threshold})
II) and control the valve device (22) in order to adjust the gas flow as a function of said comparison.

2. Apparatus according to Claim 1, **characterized in that** when the control unit (50) determines that the gas pressure (Pₘₐₛₖ) measured in the mask (10) is less than or equal to the given pressure threshold value (P_{threshold}), (i.e. Pₘₐₛₖ ≤ P_{threshold}), said control unit (50) is configured to control the valve device (22) in order to increase the flow of therapeutic gas passing through said valve device (22) and supplying the deformable reservoir (27) .

3. Apparatus according to one of the preceding claims, **characterized in that** the pressure threshold value (P_{threshold}) is less than or equal to 0 mbar.

4. Apparatus according to one of the preceding claims, **characterized in that** the pressure threshold value (P_{threshold}) is less than or equal to -0.25 mbar, preferably less than or equal to -0.5 mbar.

5. Apparatus according to one of the preceding claims, **characterized in that** the pressure threshold value (P_{threshold}) is stored in the control unit (50), preferably by the microprocessor (51) of the control unit (50).

6. Apparatus according to Claim 1, **characterized in that** the pressure sensor (55) comprises a differential pressure sensor.

7. Apparatus according to one of Claims 1 and 2, **characterized in that** the valve device (22) comprises a proportional valve.

8. Apparatus according to Claim 1, **characterized in that** it further comprises a flow sensor (60) arranged in the internal gas passage (100) in order to measure the gas flow circulating in said internal gas passage (100); preferably, the flow sensor (60) is arranged downstream of the valve device (22).

9. Apparatus according to Claim 1, **characterized in that** it further comprises a comprises a non-return device (61) arranged in the internal gas passage (100), downstream of the deformable reservoir (27).

10. Installation for supplying therapeutic gas (40) to a patient comprising a gas delivery apparatus (1) according to one of the preceding claims and a respiratory mask (10), said respiratory mask (10) being in fluid communication with the deformable reservoir (27) and supplied with therapeutic gas by said deformable reservoir (27), and also pneumatically connected to the pressure sensor (55).
